(19) Europäisches Patentamt
European Patent Office
Office européen des brevets

(11) Veröffentlichungsnummer: **0 039 081**
**B1**

(12) # EUROPÄISCHE PATENTSCHRIFT

(45) Veröffentlichungstag der Patentschrift: **15.02.84**

(21) Anmeldenummer: **81103187.1**

(22) Anmeldetag: **28.04.81**

(51) Int. Cl.³: **C 07 D 495/04,** A 61 K 31/415 // (C07D495/04, 337/00, 235/00)

(54) **Neue Imidazolderivate, ihre Herstellung, und Arzneimittel enthaltend diese Derivate.**

(30) Priorität: **29.04.80 CH 3304/80**
**16.02.81 CH 1057/81**

(43) Veröffentlichungstag der Anmeldung: **04.11.81 Patentblatt 81/44**

(45) Bekanntmachung des Hinweises auf die Patenterteilung: **15.02.84 Patentblatt 84/7**

(84) Benannte Vertragsstaaten: **AT BE CH DE FR GB IT LI LU NL SE**

(56) Entgegenhaltungen:
**Chemical Abstracts, Band 63, Nr. 5, 30. August 1965, Columbus, Ohio, USA, H. WYNBERG et al. "Synthesis of 4,5-di-tert-butylimidazole" Spalte 5630e**

(73) Patentinhaber: **F. HOFFMANN-LA ROCHE & CO. Aktiengesellschaft, CH-4002 Basel (CH)**

(72) Erfinder: **Derungs, Romano, Dr., Bäumliweg 18, CH-4125 Riehen (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 039 081 B1

ACTORUM AG

# Neue Imidazolderivate, ihre Herstellung und Arzneimittel diese Derivate enthaltend

Die vorliegende Erfindung betrifft neue Imidazolderivate der Formel

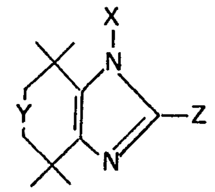

(I)

worin X Wasserstoff oder $C_{1-4}$-Alkyl, Y Schwefel oder eine Sulfinyl- oder Sulfonylgruppe und Z gegebenenfalls durch eine Methylgruppe oder ein Fluoratom substituiertes Thienyl oder eine Gruppe der Formel

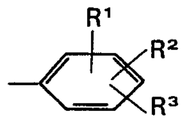

Z

ist, worin $R^1$, $R^2$ und $R^3$ Wasserstoff, Methyl, Fluor, Hydroxy, Methoxy, Methylthio oder zwei benachbarte Reste, $R^1$, $R^2$ oder $R^3$ zusammen Methylendioxy oder Äthylendioxy sind, oder einer der Reste $R^1$, $R^2$ und $R^3$ Mono- oder Di-($C_{1-4}$-Alkyl)amino ist und die zwei anderen Wasserstoff sind,
und physiologisch verträgliche Säureadditionssalze, insbesondere Mineralsäuresalze davon.

Der hier verwendete Ausdruck $C_{1-4}$-Alkyl bezieht sich auf die geradkettigen Alkylreste, Methyl, Äthyl, n-Propyl und n-Butyl.

Unter den Verbindungen der Formel I sind diejenigen bevorzugt, worin X Wasserstoff und/oder Y Schwefel und/oder Z Phenyl, p-(Fluor oder Methoxy)phenyl, 3,4,5-Trimethoxyphenyl, 3,4-Methylendioxyphenyl oder 2-Thienyl ist.

Besonders bevorzugt sind 2-Phenyl-4,5,7,8-tetrahydro-4,4,8,8-tetramethyl-1-H-thiepino-[4,5-d]imidazol und 2-p-Fluorphenyl-4,5,7,8-tetrahydro-4,4,8,8-tetramethyl-1-H-thiepino-[4,5-d]imidazol.

Die Erfindung betrifft weiterhin ein Verfahren zur Herstellung der genannten Verbindungen sowie pharmazeutische Präparate auf der Basis der genannten Verbindungen.

Beispiele physiologisch verträglicher Mineralsäuresalze sind Hydrochloride, Hydrobromide, Sulfate und Phosphate.

Die genannten Verbindungen können erfindungsgemäss dadurch hergestellt werden, dass man zur Herstellung einer Verbindung der Formel I, worin X Wasserstoff ist, ein Diketon der Formel

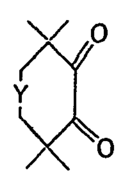

(II)

worin Y die obige Bedeutung hat,
mit einem Aldehyd der Formel

Z−CH=O (III)

worin Z die obige Bedeutung hat,
in Gegenwart eines Ammoniumionen enthaltenden polaren Lösungsmittels umsetzt, dass man zur Herstellung einer Verbindung der Formel I, worin X $C_{1-4}$-Alkyl ist, eine Verbindung der Formel I, worin X Wasserstoff ist, alkyliert; gewünschtenfalls eine Verbindung der Formel I, worin Y Schwefel ist, zu einer solchen, worin Y Sulfinyl ist, oxydiert und gewünschtenfalls eine Verbindung der Formel I in ein Säureadditionssalz überführt.

Als Quelle von Ammoniumionen werden zweckmässig Ammoniumsalze, vorzugsweise von Carbonsäuren, wie Ammoniumacetat, verwendet. Beispiele von polaren Lösungsmitteln sind DMSO und DMF. Die Reaktion des Diketons II mit dem Aldehyd III wird zweckmässig unter Erwärmen bis zur Rückflusstemperatur, vorzugsweise 50 und 120°C, insbesondere zwischen 90 und 100°C, durchgeführt.

An Stelle des Diketons II kann man das entsprechende α-Ketol als Ausgangsstoff verwenden. In diesem Fall muss die Reaktion in Gegenwart eines Oxydationsmittels, wie Kupfer (II) — oder Blei (IV) — acetat, durchgeführt werden.

Die Alkylierung einer N-unsubstituierten Verbindung I kann dadurch bewerkstelligt werden, dass man letztere mit einem Alkalimetallhydrid, z.B. NaH, in einem wasserfreien Lösungsmittel, wie Dimethylformamid (DMF), umsetzt und die erhaltene Verbindung mit einem Alkylhalogenid, z.B. Methyljodid, umsetzt.

Die Überführung einer Verbindung I, worin Y Schwefel ist, in das entsprechende Sulfoxyd kann mit einem Oxydationsmittel, wie Natriumperjodat, in einem Lösungsmittel, wie wässerigem Methanol, zweckmässig bei niedriger Temperatur, vorzugsweise bei etwa 0 bis 5°C, bewerkstelligt werden.

Die Verbindungen der Formel I und die physiologisch verträglichen Säureadditionssalze davon können als Heilmittel Verwendung finden. Sie hemmen die Aggregation der Blutplättchen und wirken antihyperglykämisch. Sie können daher zur Verhütung von Thrombosen bzw. zur Behandlung des Diabetes verwendet werden.

Die Verbindungen der Formel I können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Öle, Polyalkylenglykole oder Vaseline enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen

Druckes oder Puffer. Sie können auch noch andere therapeutisch wertvolle Stoffe enthalten. Die orale Verabreichung der erfindungsgemässen Verbindungen ist bevorzugt. Für den Erwachsenen kommen eine orale Tagesdosis von 0,5 bis 30 mg/kg und eine parenterale Tagesdosis von 0,05 bis 10 mg/kg in Frage.

Die aggregationshemmende Wirkung wurde nach der Aggregometer-Methode von BORN [Nature *194*, 927 (1962)] und MICHAL und BORN [Nature *231*, 220 (1971)] nachgewiesen. Die maximale Aggregationsgeschwindigkeit wurde als Versuchsparameter genommen und die effektive Konzentration ($EC_{50}$) aus Dosis-Wirkungskurven ermittelt.

Menschliches plättchenreiches Plasma wurde aus citriertem venösem Blut durch Zentrifugieren erhalten. Die Versuche wurden mit Suspensionen der Testsubstanzen in 0,9% NaCl durchgeführt. 0,18 ml Citratplasma wurden mit 10 µl Suspension der Testverbindungen versetzt und 10 Minuten bei 37°C inkubiert, worauf die Aggregation durch Zusatz von 10 µl einer Suspension von Kollagen-Fibrillen ausgelöst wurde.

Die Resultate sind in der Tabelle I wiedergegeben.

*Tabelle I*

*Kollageninduzierte Blutplättchenaggregation*

| Verbindung | $Ec_{50}$ (µM) |
|---|---|
| 2-Phenyl-4,5,7,8-tetrahydro-4,4,8,8,-tetramethyl-1-H-thiepino[4,5-d]imidazolhydrochlorid | 0,8 |
| 2-(3,4-Methylendioxyphenyl)-4,5,7,8-tetrahydro-4,4,8,8-tetramethyl-1-H-thiepino[4,5-d]-imidazolhydrochlorid | 1,2 |
| 2-(p-Methoxyphenyl)-4,5,7,8-tetrahydro-4,4,8,8-tetramethyl-1-H-thiepino[4,5-d]imidazol-hydrochlorid | 4,2 |
| 4,5,7,8-Tetrahydro-4,4,8,8-tetramethyl-2-(2-thienyl)-1-H-thiepino[4,5-d]imidazolhydrochlorid | 19,7 |
| 2-(p-Fluorphenyl)-4,5,7,8-tetrahydro-4,4,8,8-tetramethyl-1-H-thiepino[4,5-d]imidazolhydrochlorid | 4,7 |

Die antihyperglykämische Wirkung kann wie folgt nachgewiesen werden:

Ratten (24 Stunden gefastet) wird Gummi arabicum (12 Kontrolltiere) oder 0,3 mMol/kg p.o. Testsubstanz (6 Versuchstiere) und 100 Minuten später eine Suspension von 1,6 g Glucose in 10 ml fünfprozentigem Gummi arabicum pro kg Körpergewicht p.o. verabreicht. 20 Minuten nach der Glucosegabe werden die Tiere getötet. Aus dem heparinisierten Mischblut wird Plasma gewonnen. Die Glucosekonzentration im Plasma wird nach der Hexokinase-Methode bestimmt. Die Mittelwerte der Glucosekonzentration, ausgedrückt in Prozenten der Kontrollen, sind in der Tabelle II angegeben.

*Tabelle II*

*Plasmaglucosekonzentration*

| Verbindung | Glucose-konzentration (%) |
|---|---|
| 2-Phenyl-4,5,7,8-tetrahydro-4,4,8,8-tetramethyl-1-H-thiepino[4,5-d]imidazol | 74 |
| 2-Phenyl-4,5,7,8-tetrahydro-4,4,8,8-tetramethyl-1-H-thiepino[4,5-d]imidazol-hydrochlorid | 67 |
| 2-(p-Fluorphenyl)-4,5,7,8-tetrahydro-4,4,8,8-tetramethyl-1-H-thiepino[4,5-d]imidazol-hydrochlorid | 77 |

*Beispiel 1:*

Man löst 16 g 3,3,6,6-Tetramethyl-4,5-thiepandion und 8 g Benzaldehyd in 200 ml Dimethylsulfoxyd, fügt unter Rühren 60 g wasserfreies Ammmoniumacetat zu und erhitzt auf 90°C.

Das Reaktionsgemisch wird nach dem Abkühlen unter Rühren in Eiswasser gegossen, die Lösung mit konzentrierter Natronlauge alkalisch gestellt und mit Äther extrahiert. Die organische Phase wird mit Eiswasser gewaschen und zur Trockne eingeengt. Der Rückstand wird mit Petroläther überschichtet und mit einem Glasstab gerieben. Der abfiltrierte Niederschlag wird aus Toluol umkristallisiert. Man erhält 2-Phenyl-4,5,7,8-tetrahydro-4,4,8,8-tetramethyl-1-H-thiepino[4,5-d]imidazol, Smp. 225-227°C.

Zur Herstellung des Hydrochlorids löst man 500 mg der Base in 50 ml Äther und tropft unter Rühren ätherische Salzsäure zu, bis kein Produkt mehr ausfällt. Der Niederschlag wird abfiltiert und mit Äther und Äthanol gewaschen. Nach der Filtration und Trocknung erhält man 600 mg Salz, Smp. 300°C.

*Beispiel 2:*

In zu Beispiel 1 analoger Weise wird folgende Verbindung hergestellt:

2-(3,4-Methylendioxyphenyl)-4,5,7,8-tetrahydro-4,4,8,8-tetramethyl-1-H-thiepino[4,5-d]-imidazol, Smp. 179-180°C, Smp. des Hydrochlorids: 270°C (Zers.)

*Beispiel 3:*

4 g 3,3,6,6-Tetramethyl-4,5-thiepandion, 2,72 g Anisaldehyd und 15 g wasserfreies Ammoniumacetat werden in 50 ml Dimethylsulfoxyd gelöst. Die Reaktion wird während 4 Stunden bei 95°C unter Rühren durchgeführt.

Das Reaktionsgemisch wird auf Eiswasser gegossen und das Gemisch mit Ammoniak alkalisch gestellt. Dann wird mit Äther extrahiert. Die vereinigten Extrakte werden mit Wasser gewaschen, getrocknet und vom Lösungsmittel befreit. Der Rückstand wird in Äther gelöst und mit Petroläther bis zur beginnenden Trübung versetzt. Nach dem Auskristallisieren werden die Kristalle abfiltriert und mit Äther gewaschen. Nach Umkristallisation aus n-Heptan erhält man 2-(p-Methoxyphenyl)-4,5,7,8-tetrahydro-4,4,8,8-tetramethyl-1-H-thiepino[4,5-d]imidazol, Smp. 175-176°C, Smp. des Hydrochlorids: 250°C (Zers.).

*Beispiel 4:*

In zu Beispiel 3 analoger Weise werden folgende Verbindungen hergestellt:

2-(m-Methoxyphenyl-4,5,7,8-tetrahydro-4,4,8,8-tetramethyl-1-H-thiepino[4,5-d]imidazol, Smp. 185-187°C, Smp. des Hydrochlorids: 280°C (Zers.),

2-(o-Methoxyphenyl)-4,5,7,8-tetrahydro-4,4,8,8-tetramethyl-1-H-thiepino[4,5-d]imidazol, Smp. 140-141°C, Smp. des Hydrochlorids: 245°C (Zers.),

4,5,7,8-Tetrahydro-4,4,8,8-tetramethyl-2-p-tolyl-1-H-thiepino[4,5-d]imidazol, Smp. 211-213°C, Smp. des Hydrochlorids: 270-275°C,

4,5,7,8-Tetrahydro-4,4,8,8-tetramethyl-2-m-tolyl-1-H-thiepino[4,5-d]imidazol, Smp. 237-240°C, Smp. des Hydrochlorids: 260°C (Zers.),

2-(p-Methylthiophenyl)-4,5,7,8-tetrahydro-4,4,8,8-tetramethyl-1-H-thiepino[4,5-d]imidazol, Smp. 165-167°C, Smp. des Hydrochlorids: 270-280°C (Zers.),

4,5,7,8-Tetrahydro-4,4,8,8-tetramethyl-2-o-tolyl-1-H-thiepino[4,5-d]imidazol, Smp. 110-115°C, Smp. des Hydrochlorids: 275°C (Zers.),

p-(4,5,7,8-Tetrahydro-4,4,8,8-tetramethyl-1-H-thiepino[4,5-d]imidazol-2-yl)phenol, Smp. 255°C, Smp. des Hydrochlorids: 300°C (Zers.),

m-(4,5,7,8-Tetrahydro-4,4,8,8-tetramethyl-1-H-thiepino[4,5-d]imidazol-2-yl)phenol, Smp. 118-120°C, Smp. des Hydrochlorids: 300°C (Zers.),

o-(4,5,7,8-Tetrahydro-4,4,8,8-tetramethyl-1-H-thiepino[4,5-d]imidazol-2-yl)phenol, Smp. 204-206°C, Smp. des Hydrochlorids: 250°C (Zers.),

2-(4,6-Dimethylphenyl)-4,5,7,8-tetrahydro-4,4,8,8-tetramethyl-1-H-thiepino[4,5-d]imidazol, Smp. 142-144°C, Smp. des Hydrochlorids: 310°C (Zers.),

2-(3,4-Dimethylphenyl)-4,5,7,8-tetrahydro-4,4,8,8-tetramethyl-1-H-thiepino[4,5-d]imidazol, Smp. 218-220°C, Smp. des Hydrochlorids: 275°C (Zers.),

2-(3,4-Dimethoxyphenyl)-4,5,7,8-tetrahydro-4,4,8,8-tetramethyl-1-H-thiepino[4,5-d]imidazol, Smp. 138-140°C, Smp. des Hydrochlorids: 140°C (Zers.),

4,5,7,8-Tetrahydro-4,4,8,8-tetramethyl-2-(2,4,5-trimethoxyphenyl)-1-H-thiepino[4,5-d]-imidazol, Smp. 141-143°C, Smp. des Hydrochlorids: 218-220°C,

4,5,7,8-Tetrahydro-4,4,8,8-tetramethyl-2-(2,4,6-trimethoxyphenyl)-1-H-thiepino[4,5-d]-imidazol, Smp. 141-142°C, Smp. des Hydrochlorids: 235-240°C (Zers.),

4,5,7,8-Tetrahydro-4,4,8,8-tetramethyl-2-(3,4,5-trimethoxyphenyl)-1-H-thiepino[4,5-d]-imidazol, Smp. 188-190°C, Smp. des Hydrochlorids: 290°C (Zers.),

2-(3,4-Äthylendioxyphenyl)-4,5,7,8-tetrahydro-4,4,8,8-tetramethyl-1-H-thiepino[4,5-d]-imidazol, Smp. 178-180°C, Smp. des Hydrochlorids: 195°C (Zers.),

2-[p-(Diäthylamino)phenyl]-4,5,7,8-tetrahydro-4,4,8,8-tetramethyl-1-H-thiepino[4,5-d]-imidazol, Smp. 190-192°C, Smp. des Hydrochlorids: 250°C (Zers.),

4,5,7,8-Tetrahydro-4,4,8,8-tetramethyl-2-(2-thienyl)-1-H-thiepino[4,5-d]imidazol, Smp. 213-215°C, Smp. des Hydrochlorids: 250°C (Zers.),

2-(m-Fluorphenyl)-4,5,7,8-tetrahydro-4,4,8,8-tetramethyl-1-H-thiepino[4,5-d]imidazol, Smp. 199-200°C, Smp. des Hydrochlorids: 290°C (Zers.),

2-(o-Fluorphenyl)-4,5,7,8-tetrahydro-4,4,8,8-tetramethyl-1-H-thiepino[4,5-d]imidazol, Smp. 98-99°C, Smp. des Hydrochlorids: 300°C (Zers.),

2-(p-Fluorphenyl)-4,5,7,8-tetrahydro-4,4,8,8-tetramethyl-1-H-thiepino[4,5-d]imidazol, Smp. 195-198°C, Smp. des Hydrochlorids: 300°C (Zers.).

*Beispiel 5:*

4g 3,3,6,6-Tetramethyl-4,5-thiepandion und 3,6 g 3,4-Methylendioxy-5-methoxybenzaldehyd werden in 50 ml Dimethylformamid gelöst. Dann gibt man 15,5 g wasserfreies Ammoniumacetat zu. Die Reaktion wird unter Rühren während 5 Stunden bei 95°C durchgeführt.

Nach dem Abkühlen wird unter Rühren die Reaktionsmischung auf Eiswasser gegossen. Die Mischung wird mit Ammoniak alkalisch gestellt, dann mit Äther extrahiert. Die organische Phase wird mit Wasser gewaschen, getrocknet und eingeengt. Der kristalline Rückstand wird mit Äther und Petroläther zerrieben und abgenutscht. Nach Umkristallisation aus n-Heptan erhält man 2-(5-Methoxy-3,4-methylendioxyphenyl)-4,5,7,8-tetrahydro-4,4,8,8-tetramethyl-1-H-thiepino-[4,5-d]imidazol, Smp. 175-178°C, Smp. des Hydrochlorids: 300°C (Zers.).

*Beispiel 6:*

1,5 g 3,3,6,6-Tetramethyl-4,5-thiepandion-1,1-dioxyd, 7 g Benzaldehyd und 5 g wasserfreies Ammoniumacetat werden in 16 ml Dimethylsulfoxyd gelöst. Die Reaktionsmischung wird unter Rühren auf 95°C erwärmt und anschliessend lässt man unter den selben Bedingungen noch 4 Stunden reagieren. Bei Zimmertemperatur wird dann das Gemisch unter Rühren auf Eiswasser gegossen, mit konzentrierter Natronlauge wird alkalisch gestellt und mit Essigester extrahiert. Die organische Phase wird mit Eiswasser gewaschen und getrocknet. Das Lösungsmittel wird abdestilliert. Der Rückstand wird mit Äther versetzt, wobei das Produkt auskristallisiert. Zur Umkristallisation wird in Essigester gelöst und nach der Filtration mit n-Hexan versetzt. Man erhält 2-Phenyl-4,5,7,8-tetrahydro-4,4,8,8-tetramethyl-1-H-thiepino-[4,5-d]imidazol-6,6-dioxyd, Smp. 260-263°C, Smp. des Hydrochlorids: 300°C.

*Beispiel 7:*

In zu Beispiel 6 analoger Weise wird hergestellt das 2-(m-Methoxyphenyl)-4,5,7,8-tetrahydro-4,4,8,8-tetramethyl-1-H-thiepino[4,5-d]imidazol-6,6-dioxyd, Smp. 217-219°C.

*Beispiel 8:*

4,04 g 5-Hydroxy-2,3,6,7-tetrahydro-3,3,6,6-tetramethyl-4(5H)-thiepinon werden unter Rühren und bei 50°C in 100 ml Methanol gelöst. 5 g Kupfer(II)-acetatmonohydrat werden zugegeben, gefolgt von 2,12 g Benzaldehyd. Dann werden 60 ml konz. wässerige Ammoniaklösung zugetropft. Dann wird 4 Stunden am Rückfluss gekocht und abschliessend die Reaktionsmischung heiss abfiltriert. Der Nutschkuchen wird mit heissem Methanol aufgeschwemmt und dann trockengesaugt.

Der Kupfersalz-Niederschlag wird in wässerigem Äthanol suspendiert und mit 2N Salzsäure sauer gestellt. Dann wird unter Rühren bei 80°C Schwefelwasserstoff eingeleitet. Nach 3 Stunden wird vom Kupfersulfid abfiltriert. Das Filtrat wird eingeengt, die Suspension mit konz. Ammoniak alkalisch gestellt und mit Äther extrahiert. Nach dem Trocknen der organischen Phase und Einengen wird der Rückstand aus n-Heptan umkristallisiert. Man erhält 2-Phenyl-4,5,7,8-tetrahydro-4,4,8,8-tetramethyl-1-H-thiepino[4,5-d]imidazol, Smp. 223-224°C.

*Beispiel 9:*

1,43 g 2-Phenyl-4,5,7,8-tetrahydro-4,4,8,8-tetramethyl-1-H-thiepino[4,5-d]imidazol werden in 75 ml Methanol gelöst und unter Rühren auf 2°C abgekühlt. Die Lösung wird bei dieser Temperatur mit einer Lösung von 1,3 g Natriumperjodat in 28 ml Wasser betropft. Man lässt bei gleicher Temperatur während 3 Stunden ausreagieren. Hierauf werden 25 ml Methanol zugegeben. Nach 5 Stunden Rühren bei Raumtemperatur wird die Lösung eingeengt. Man lässt das Produkt unter Eiskühlung auskristallisieren. Der Niederschlag wird abfiltriert und die Kristalle mit Aceton und Äther überschichtet. Nach der Filtration wird das 2-Phenyl-4,5,7,8-tetrahydro-4,4,8,8-tetramethyl-1-H-thiepino[4,5-d]imidazol-6-oxyd aus Äthanol/n-Hexan umkristallisiert, Smp. 273-275°C, Smp. des Hydrochlorids: 300°C.

*Beispiel 10:*

In zu Beispiel 9 analoger Weise wird hergestellt 2-(m-Methoxyphenyl)-4,5,7,8-tetrahydro-4,4,8,8-tetramethyl-1-H-thiepino[4,5-d]imidazol-6-oxyd, Smp. 205-207°C.

*Beispiel 11:*

Unter Strickstoff oder Argon wird eine Suspension von 0,44 g Natriumhydrid (55% in Paraffin) in 10 ml Dimethylformamid auf 0°C abgekühlt. Hierauf wird eine Lösung von 2,86 g 2-Phenyl-4,5,7,8-tetrahydro-4,4,8,8-tetramethyl-1-H-thiepino[4,5-d]imidazol in 10 ml Dimethylformamid zugetropft. Dann lässt man 20 Minuten bei Raumtemperatur ausreagieren. Danach werden 2,1 g Methyljodid in 10 ml Dimethylformamid zugetropft. Man lässt noch 30 Minuten bei Raumtemperatur ausreagieren.

Die Reaktionsmischung wird unter Rühren auf Eiswasser gegossen, der Niederschlag abfiltriert und in Wasser gewaschen. Der Filterrückstand wird in Äther gelöst und die Lösung getrocknet und eingeengt. Die Suspension wird mit Petroläther versetzt. Nach der Kristallisation wird filtriert und das Produkt aus n-Heptan umkristallisiert. Das 2-Phenyl-4,5,7,8-tetrahydro-1,4,4,8,8-pentamethyl-1-H-thiepino[4,5-d]imidazol schmilzt bei 158-160°C. Smp. des Hydrochlorids: 240°C (Zers.).

*Beispiel 12:*

In zu Beispiel 11 analoger Weise werden folgende Verbindungen hergestellt:

2-Phenyl-4,5,7,8-tetrahydro-1,4,4,8,8-pentamethyl-1-H-thiepino[4,5-d]imidazol-6-oxid, Smp. 178-180°C, Smp. des Hydrochlorids: 170°C (Zers.),

1-n-Butyl-2-phenyl-4,5,7,8-tetrahydro-4,4,8,8-tetramethyl-1-H-thiepino[4,5-d]imidazol, Smp. 100°C, Smp. des Hydrochlorids: 120°C (Zers.).

*Beispiel 13:*

In üblicher Weise werden Tabletten folgender Zusammensetzung hergestellt:

| | |
|---|---|
| 2-p-Fluorphenyl-4,5,7,8-tetrahydro-4,4,8,8-tetramethyl-1-H-thiepino-[4,5-d]imidazolhydrochlorid | 185,0 mg |
| Milchzucker | 15,0 mg |
| Maisstärke | 37,9 mg |
| Wasserlösliches Polyvinylpyrrolidon | 10,0 mg |
| Magnesiumstearat | 2,5 mg |
| Gesamtgewicht pro Tablette | 250,0 mg |

*Beispiel 14:*

In üblicher Weise werden Gelatinesteckkapseln folgender Zusammensetzung hergestellt:

| | |
|---|---|
| 2-p-Fluorphenyl-4,5,7,8-tetrahydro-4,4,8,8-tetramethyl-1-H-thiepino-[4,5-d]imidazolhydrochlorid | 200,0 mg |
| Wasserlösliche Polyvinylpyrrolidon | 2,0 mg |
| Maisstärke | 43,0 mg |
| Talk | 4,5 mg |
| Magnesiumstearat | 0,5 mg |
| Gesamtgewicht pro Kapsel | 250,0 mg |

*Beispiel 15:*

In üblicher Weise wird eine Injektionslösung folgender Zusammensetzung hergestellt:

2-p-Fluorphenyl-4,5,7,8-tetrahydro-

| | |
|---|---|
| 4,4,8,8-tetramethyl-1-H-thiepino-[4,5-d]imidazolhydrochlorid | 115,0 mg |
| Glycerinformal | 2,4 ml |
| Wasser | 4,9 ml |

**Patentansprüche für die Vertragsstaaten:** BE, CH, DE, FR, GB, IT, LI, LU, NL, SE

1. Imidazolderivate der Formel

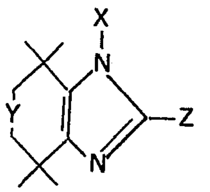

(I)

worin X Wasserstoff oder $C_{1-4}$-Alkyl, Y Schwefel oder eine Sulfinyl- oder Sulfonylgruppe und Z gegebenenfalls durch eine Methylgruppe oder ein Fluoratom substituiertes Thienyl oder eine Gruppe der Formel

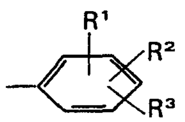

Z

ist, worin $R^1$, $R^2$ und $R^3$ Wasserstoff, Methyl, Fluor, Hydroxy, Methoxy, Methylthio, oder zwei benachbarte Reste $R^1$, $R^2$ oder $R^3$ zusammen Methylendioxy oder Äthylendioxy sind oder einer der Reste $R^1$, $R^2$ und $R^3$ Mono- oder Di-($C_{1-4}$-alkyl)amino ist und die zwei anderen Wasserstoff sind,
und physiologisch verträgliche Säureadditionssalze davon.

2. Verbindungen nach Anspruch 1, worin X Wasserstoff ist.

3. Verbindungen nach Anspruch 1 oder 2, worin Y Schwefel ist.

4. Verbindungen nach Anspruch 1, 2 oder 3, worin Z Phenyl, p-(Fluor oder Methoxy)phenyl, 3,4,5-Trimethoxyphenyl, 3,4-Methylendioxyphenyl oder 2-Thienyl ist.

5. 2-Phenyl-4,5,7,8-tetrahydro-4,4,8,8-tetramethyl-1-H-thiepino[4,5-d]imidazol.

6. 2-p-Fluorphenyl-4,5,7,8-tetrahydro-4,4,8,8-tetramethyl-1-H-thiepino[4,5-d]imidazol.

7. Die Verbindungen 2-(p-Methoxyphenyl)-4,5,7,8-tetrahydro-4,4,8,8-tetramethyl-1-H-thiepino[4,5-d]imidazol,
2-(m-Methoxyphenyl)-4,5,7,8-tetrahydro-4,4,8,8-tetramethyl-1-H-thiepino[4,5-d]imidazol,
2-(o-Methoxyphenyl)-4,5,7,8-tetrahydro-4,4,8,8-tetramethyl-1-H-thiepino[4,5-d]imidazol,
2-(3,4-Dimethoxyphenyl)-4,5,7,8-tetrahydro-4,4,8,8-tetramethyl-1-H-thiepino[4,5-d]imidazol,
4,5,7,8-Tetrahydro-4,4,8,8-tetramethyl-2-(2,4,5-trimethoxyphenyl)-1-H-thiepino[4,5-d]-imidazol,
4,5,7,8-Tetrahydro-4,4,8,8-tetramethyl-2-(2,4,6-trimethoxyphenyl)-1-H-thiepino[4,5-d]-imidazol,
4,5,7,8-Tetrahydro-4,4,8,8-tetramethyl-2-(3,4,5-trimethoxyphenyl)-1-H-thiepino[4,5-d]-imidazol,
2-(m-Methoxyphenyl)-4,5,7,8-tetrahydro-4,4,8,8-tetramethyl-1-H-thiepino[4,5-d]imidazol-6,6-dioxyd,
2-(m-Methoxyphenyl)-4,5,7,8-tetrahydro-4,4,8,8-tetramethyl-1-H-thiepino[4,5-d]imidazol-6-oxyd.

8. Die Verbindungen 4,5,7,8-Tetrahydro-4,4,8,8-tetramethyl-2-p-tolyl-1-H-thiepino-[4,5-d]imidazol,
4,5,7,8-Tetrahydro-4,4,8,8-tetramethyl-2-m-tolyl-1-H-thiepino[4,5-d]imidazol,
4,5,7,8-Tetrahydro-4,4,8,8-tetramethyl-2-o-tolyl-1-H-thiepino[4,5-d]imidazol,
2-(4,6-Dimethylphenyl)-4,5,7,8-tetrahydro-4,4,8,8-tetramethyl-1-H-thiepino[4,5-d]imidazol,
2-(3,4-Dimethylphenyl)-4,5,7,8-tetrahydro-4,4,8,8-tetramethyl-1-H-thiepino[4,5-d]imidazol.

9. Die Verbindungen p-(4,5,7,8-Tetrahydro-4,4,8,8-tetramethyl-1-H-thiepino[4,5-d]imidazol-2-yl)phenol,
m-(4,5,7,8-Tetrahydro-4,4,8,8-tetramethyl-1-H-thiepino[4,5-d]imidazol-2-yl)phenol,
o-(4,5,7,8-Tetrahydro-4,4,8,8-tetramethyl-1-H-thiepino[4,5-d]imidazol-2-yl)phenol.

10. Die Verbindungen 2-(3,4-Methylendioxyphenyl)-4,5,7,8-tetrahydro-4,4,8,8-tetramethyl-1-H-thiepino[4,5-d]imidazol,
2-(3,4-Äthylendioxyphenyl)-4,5,7,8-tetrahydro-4,4,8,8-tetramethyl-1-H-thiepino[4,5-d]imidazol,
2-(5-Methoxy-3,4-methylendioxyphenyl)-4,5,7,8-tetrahydro-4,4,8,8-tetramethyl-1-H-thiepino[4,5-d]imidazol.

11. Die Verbindungen 2-(m-Fluorphenyl)-4,5,7,8-tetrahydro-4,4,8,8-tetramethyl-1-H-thiepino[4,5-d]imidazol,
2-(p-Fluorphenyl)-4,5,7,8-tetrahydro-4,4,8,8-tetramethyl-1-H-thiepino[4,5-d]imidazol.

12. Die Verbindungen 2-Phenyl-4,5,7,8-tetrahydro-4,4,8,8-tetramethyl-1-H-thiepino[4,5-d]-imidazol-6,6-dioxyd,
2-Phenyl-4,5,7,8-tetrahydro-4,4,8,8-tetramethyl-1-H-thiepino[4,5-d]imidazol-6-oxyd,
2-Phenyl-4,5,7,8-tetrahydro-1,4,4,8,8-pentamethyl-1-H-thiepino[4,5-d]imidazol,
2-Phenyl-4,5,7,8-tetrahydro-1,4,4,8,8-pentamethyl-1-H-thiepino[4,5-d]imidazol-6-oxyd,
1-n-Butyl-2-phenyl-4,5,7,8-tetrahydro-4,4,8,8-tetramethyl-1-H-thiepino[4,5-d]imidazol.

13. 2-[p-(Diäthylamino)phenyl]-4,5,7,8-tetrahydro-4,4,8,8-tetramethyl-1-H-thiepino-[4,5-d]imidazol.

14. 2-(p-Methylthiophenyl)-4,5,7,8-tetrahydro-4,4,8,8-tetramethyl-1-H-thiepino[4,5-d]-imidazol.

15. 4,5,7,8-Tetrahydro-4,4,8,8-tetramethyl-2-(2-thienyl)-1-H-thiepino[4,5-d]imidazol.

16. Eine Verbindung nach einem der Ansprüche 1-15 als pharmazeutischer Wirkstoff.

17. Eine Verbindung nach einem der Ansprüche 1-15 als die Blutplättchenaggregation hemmender Wirkstoff.

18. Eine Verbindung nach einem der Ansprüche 1-15 als antihyperglykämischer Wirkstoff.

19. Heilmittel enthaltend eine Verbindung nach einem der Ansprüche 1-15.

20. Mittel zur Hemmung der Blutplättchenaggregation, enthaltend eine Verbindung nach einem der Ansprüche 1-15.

21. Antihyperglykämisches Mittel, enthaltend eine Verbindung nach einem der Ansprüche 1-15.

22. Verfahren zur Herstellung von Verbindungen der Formel I in Anspruch 1 und von physiologisch verträglichen Säureadditionssalzen, dadurch gekennzeichnet, dass man zur Herstellung einer Verbindung der Formel I worin X Wasserstoff ist, ein Diketon der Formel

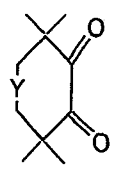

(II)

worin Y die obige Bedeutung hat,
mit einem Aldehyd der Formel

$$Z-CH=O \qquad (III)$$

worin Z die obige Bedeutung hat,
in Gegenwart eines Ammoniumionen enthaltenden polaren Lösungsmittels umsetzt; dass man zur Herstellung einer Verbindung der Formel I, worin X $C_{1-4}$-Alkyl ist, eine Verbindung der Formel I, worin X Wasserstoff ist, alkyliert; gewünschtenfalls eine Verbindung der Formel I, worin Y Schwefel ist zu einer solchen, worin Y Sulfinyl ist, oxydiert und gewünschtenfalls eine Verbindung der Formel I in ein physiologisch verträgliches Säureadditionssalz überführt.

**Patentansprüche für den Vertragsstaat:** AT

1. Verfahren zur Herstellung von Imidazolderivaten der Formel

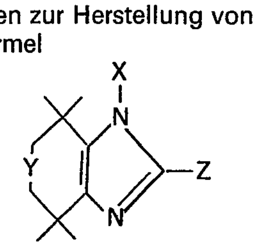

(I)

worin X Wasserstoff oder $C_{1-4}$-Alkyl, Y Schwefel oder eine Sulfinyl- oder Sulfonylgruppe und Z gegebenenfalls durch eine Methylgruppe oder ein Fluoratom substituiertes Thienyl oder eine Gruppe der Formel

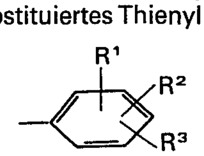

Z

ist, worin $R^1$, $R^2$ und $R^3$ Wasserstoff, Methyl, Fluor, Hydroxy, Methoxy, Methylthio, oder zwei benachbarte Rerste $R^1$, $R^2$ und $R^3$ zusammen Methylendioxy oder Äthylendioxy sind oder einer der Reste $R^1$, $R^2$ und $R^3$ Mono- oder Di-($C_{1-4}$-alkyl)amino ist und die zwei anderen Wasserstoff sind,
und von physiologisch verträglichen Säureadditionssalzen davor, dadurch gekennzeichnet, dass man zur Herstellung einer Verbindung der Formel I worin X Wasserstoff ist, ein Diketon der Formel

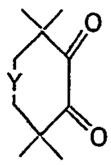

(II)

worin Y die obige Bedeutung hat,
mit einem Aldehyd der Formel

$$Z-CH=O \qquad (III)$$

worin Z die obige Bedeutung hat,
in Gegenwart eines Ammoniumionen enthaltenden polaren Lösungsmittels umsetzt, dass man zur Herstellung einer Verbindung der Formel I, worin X $C_{1-4}$-Alkyl ist, eine Verbindung der Formel I, worin X Wasserstoff ist, alkyliert; gewünschtenfalls eine Verbindung der Formel I, worin Y Schwefel ist zu einer solchen, worin Y Sulfinyl ist, oxydiert und gewünschtenfalls eine Verbindung der Formel I in ein physiologisch verträgliches Säureadditionssalz überführt.

2. Verfahren nach Anspruch 1, dadurch gekennzeichnet, dass man als Quelle von Ammoniumionen ein Ammoniumsalz verwendet.

3. Verfahren nach Anspruch 1 oder 2, dadurch gekennzeichnet, dass man Verbindungen der Formel I herstellt, in denen X Wasserstoff ist.

4. Verfahren nach Anspruch 1, 2, oder 3, dadurch gekennzeichnet, dass man Verbindungen der Formel I herstellt, in denen Y Schwefel ist.

5. Verfahren nach einem der Ansprüche 1-4, dadurch gekennzeichnet, dass man Verbindungen der Formel I herstellt, in denen Z Phenyl, p-(Fluor oder Methoxy)phenyl, 3,4,5-Trimethoxyphenyl, 3,4-Methylendioxyphenyl oder 2-Thienyl ist.

6. Verfahren nach Anspruch 5, dadurch gekennzeichnet, dass man 2-Phenyl-4,5,7,8-tetrahydro-4,4,8,8-tetramethyl-1-H-thiepino[4,5-d]imidazol herstellt.

7. Verfahren nach Anspruch 5, dadurch gekennzeichnet, dass man 2-p-Fluorphenyl-4,5,7,8-tetrahydro-4,4,8,8-tetramethyl-1-H-thiepino-[4,5-d]imidazol herstellt.

**Claims for the Contracting States:** BE, CH, DE, FR, GB, IT, LI, LU, NL, SE

1. Imidazole derivatives of the formula

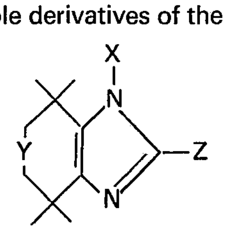

(I)

wherein X is hydrogen or $C_{1-4}$-alkyl, Y is sulphur or a sulphinyl or sulphonyl group and Z is thienyl, optionally substituted by a methyl group or a fluorine atom, or a group of the formula

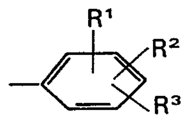

Z

wherein $R^1$, $R^2$ and $R^3$ are hydrogen, methyl, fluorine, hydroxy, methoxy, methylthio, or two adjacent residues $R^1$, $R^2$ or $R^3$ are together methylenedioxy or ethylenedioxy or one of the residues $R^1$, $R^2$ and $R^3$ is mono- or di-($C_{1-4}$-alkyl)amino and the other two are hydrogen,
and physiologically compatible acid addition salts thereof.

2. Compounds according to claim 1, wherein X is hydrogen.

3. Compounds according to claim 1 or 2, wherein Y is sulphur.

4. Compounds according to claim 1, 2 or 3, wherein Z is phenyl, p-(fluoro or methoxy)-phenyl, 3,4,5-trimethoxyphenyl, 3,4-methylenedioxyphenyl or 2-thienyl.

5. 2-Phenyl-4,5,7,8-tetrahydro-4,4,8,8-tetramethyl-1-H-thiepino[4,5-d]imidazole.

6. 2-p-Fluorophenyl-4,5,7,8-tetrahydro-4,4,8,8-tetramethyl-1-H-thiepino[4,5-d]-imidazole.

7. The compounds 2-(p-methoxyphenyl)-4,5,7,8-tetrahydro-4,4,8,8-tetramethyl-1-H-thiepino[4,5-d]imidazole,
2-(m-methoxyphenyl)-4,5,7,8-tetrahydro-4,4,8,8-tetramethyl-1-H-thiepino[4,5-d]-imidazole,
2-(o-methoxyphenyl)-4,5,7,8-tetrahydro-4,4,8,8-tetramethyl-1-H-thiepino[4,5-d]-imidazole,
2-(3,4-dimethoxyphenyl)-4,5,7,8-tetrahydro-4,4,8,8-tetramethyl-1-H-thiepino[4,5-d]-imidazole,
4,5,7,8-tetrahydro-4,4,8,8-tetramethyl-2-(2,4,5-trimethoxyphenyl)-1-H-thiepino[4,5-d]imidazole,
4,5,7,8-tetrahydro-4,4,8,8-tetramethyl-2-(2,4,6-trimethoxyphenyl)-1-H-thiepino[4,5-d]imidazole,
4,5,7,8-tetrahydro-4,4,8,8-tetramethyl-2-(3,4,5-trimethoxyphenyl)-1-H-thiepino[4,5-d]imidazole,
2-(m-methoxyphenyl)-4,5,7,8-tetrahydro-4,4,8,8-tetramethyl-1-H-thiepino[4,5-d]imidazole 6,6-dioxide,
2-(m-methoxyphenyl)-4,5,7,8-tetrahydro-4,4,8,8-tetramethyl-1-H-thiepino[4,5-d]imidazole 6-oxide.

8. The compounds 4,5,7,8-tetrahydro-4,4,8,8-tetramethyl-2-p-tolyl-1-H-thiepino[4,5-d]imidazole,
4,5,7,8-tetrahydro-4,4,8,8-tetramethyl-2-m-tolyl-1-H-thiepino[4,5-d]imidazole,
4,5,7,8-tetrahydro-4,4,8,8-tetramethyl-2-o-tolyl-1-H-thiepino[4,5-d]imidazole,
2-(4,6-dimethylphenyl)-4,5,7,8-tetrahydro-4,4,8,8-tetramethyl-1-H-thiepino[4,5-d]imidazole,
2-(3,4-dimethylphenyl)-4,5,7,8-tetrahydro-4,4,8,8-tetramethyl-1-H-thiepino[4,5-d]imidazole.

9. The compounds p-(4,5,7,8-tetrahydro-4,4,8,8-tetramethyl-1-H-thiepino[4,5-d]imidazol-2-yl)phenol,
m-(4,5,7,8-tetrahydro-4,4,8,8-tetramethyl-1-H-thiepino[4,5-d]imidazol-2-yl)phenol,
o-(4,5,7,8-tetrahydro-4,4,8,8-tetramethyl-1-H-thiepino[4,5-d]imidazol-2-yl)phenol.

10. The compounds 2-(3,4-methylenedioxyphenyl)-4,5,7,8-tetrahydro-4,4,8,8-tetramethyl-1-H-thiepino[4,5-d]imidazole,
2-(3,4-ethylenedioxyphenyl)-4,5,7,8-tetrahydro-4,4,8,8-tetramethyl-1-H-thiepino[4,5-d]-imidazole,
2-(5-methoxy-3,4-methylenedioxyphenyl)-4,5,7,8-tetrahydro-4,4,8,8-tetramethyl-1-H-thiepino[4,5-d]imidazole.

11. The compounds 2-(m-fluorophenyl)-4,5,7,8-tetrahydro-4,4,8,8-tetramethyl-1-H-thiepino[4,5-d]imidazole,
2-(p-fluorophenyl)-4,5,7,8-tetrahydro-4,4,8,8-tetramethyl-1-H-thiepino[4,5-d]imidazole.

12. The compounds 2-phenyl-4,5,7,8-tetrahydro-4,4,8,8-tetramethyl-1-H-thiepino[4,5-d] imidazole 6,6-dioxide,
2-phenyl-4,5,7,8-tetrahydro-4,4,8,8-tetrameth-yl-1-H-thiepino[4,5-d]imidazole 6-oxide,
2-phenyl-4,5,7,8-tetrahydro-1,4,4,8,8-pentamethyl-1-H-thiepino[4,5-d]imidazole,
2-phenyl-4,5,7,8-tetrahydro-1,4,4,8,8-pentamethyl-1-H-thiepino[4,5-d]imidazole 6-oxide,
1-n-butyl-2-phenyl-4,5,7,8-tetrahydro-4,4,8,8-tetramethyl-1-H-thiepino[4,5-d]imidazole.

13. 2-[p-(Diethylamino)phenyl]-4,5,7,8-tetrahydro-4,4,8,8-tetramethyl-1-H-thiepino-[4,5-d]imidazole.

14. 2-(p-Methylthiophenyl)-4,5,7,8-tetrahydro-4,4,8,8-tetramethyl-1-H-thiepino[4,5-d]-imidazole.

15. 4,5,7,8-Tetrahydro-4,4,8,8-tetramethyl-2-(2-thienyl)-1-H-thiepino[4,5-d]imidazole.

16. A compound according to any one of claims 1-15 as a pharmaceutically active substance.

17. A compound according to any one of claims 1-15 as an active substance which inhibits blood platelet aggregation.

18. A compound according to any ond of claims 1-15 as an antihyperglycaemic active substance.

19. Medicament containing a compound according to any one of claims 1-15.

20. Medicament for inhibiting blood platelet aggregation, containing a compound according to any one of claims 1-15.

21. Antihyperglycaemic medicament, containing a compound according to any one of claims 1-15.

22. Process for the manufacture of compounds of formula I in claim 1 and of physiologically

compatible acid addition salts thereof, characterized in that for the manufacture of a compound of formula I wherein X is hydrogen a diketone of the formula

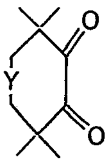

(II)

wherein Y has the above significance,
is reacted with an aldehyde of the formula

$$Z-CH=O \quad (III)$$

wherein Z has the above significance,
in the presence of a polar solvent containing ammonium ions; in that for the manufacture of a compound of formula I wherein X is $C_{1-4}$-alkyl a compound of formula I wherein X is hydrogen is alkylated; if desired, a compound of formula I wherein Y is sulphur is oxidized to a compound of formula I wherein Y is sulphinyl, and, if desired, a compound of formula I is converted into a physiologically compatible acid addition salt.

**Claims for the Contracting State:** AT

1. Process for the manufacture of imidazole derivatives of the formula

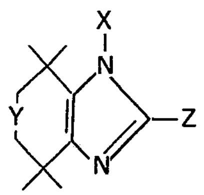

(I)

wherein X is hydrogen or $C_{1-4}$-alkyl, Y is sulphur or a sulphinyl or sulphonyl group and Z is thienyl, optionally substituted by a methyl group or a fluorine atom, or a group of the formula

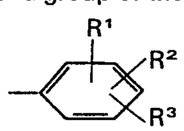

Z

wherein $R^1$, $R^2$ and $R^3$ are hydrogen, methyl, fluorine, hydroxy, methoxy, methylthio, or two adjacent residues $R^1$, $R^2$ and $R^3$ are together methylenedioxy or ethylenedioxy or one of the residues $R^1$, $R^2$ and $R^3$ is mono- or di-($C_{1-4}$-alkyl)amino and the other two are hydrogen,
and of physiologically compatible acid addition salts thereof, characterized in that for the manufacture of a compound of formula I wherein X is hydrogen a diketone of the formula

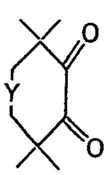

(II)

wherein Y has the above significance,
is reacted with an aldehyde of the formula

$$Z-CH=O \quad (III)$$

wherein Z has the above significance,
in the presence of a polar solvent containing ammonium ions, in that for the manufacture of a compound of formula I wherein X is $C_{1-4}$-alkyl a compound of formula I wherein X is hydrogen is alkylated; if desired, a compound of formula I wherein Y is sulphur is oxidized to a compound of formula I wherein Y is sulphinyl, and, if desired, a compound of formula I is converted into a physiologically compatible acid addition salt.

2. Process according to claim 1, characterized in that an ammonium salt is used as the source of ammonium ions.

3. Process according to claim 1 or 2, characterized in that compounds of formula I in which X is hydrogen are manufactured.

4. Process according to claim 1, 2 or 3, characterized in that compounds of formula I in which Y is sulphur are manufactured.

5. Process according to any one of claims 1-4, characterized in that compounds of formula I in which Z is phenyl, p-(fluoro or methoxy)phenyl, 3,4,5-trimethoxyphenyl, 3,4-methylenedioxyphenyl or 2-thienyl are manufactured.

6. Process according to claim 5, characterized in that 2-phenyl-4,5,7,8-tetrahydro-4,4,8,8-tetramethyl-1-H-thiepino[4,5-d]imidazole is manufactured.

7. Process according to claim 5, characterized in that 2-p-fluorophenyl-4,5,7,8-tetrahydro-4,4,8,8-tetramethyl-1-H-thiepino[4,5-d]imidazole is manufactured.

**Revendications pour les Etats contractants:** BE, CH, DE, FR, GB, IT, LI, LU, NL, SE

1. Dérivés d'imidazole de formule:

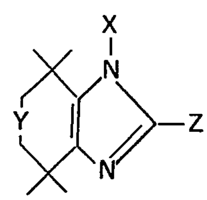

(I)

où X représente un hydrogène ou un alcoyle en $C_1$ à $C_4$, Y représente un soufre ou un groupe sulfinyle ou sulfonyle et Z un thiényle éventuellement substitué par un groupe méthyle ou un atome de fluor, ou un groupe de formule:

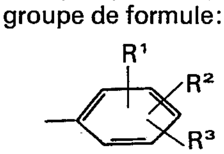

Z

où $R^1$, $R^2$ et $R^3$ représentent un hydrogène, un méthyle, un fluor, un hydroxy, un méthoxy, un méthylthio, ou bien deux radicaux $R^1$, $R^2$ ou $R^3$ voisins représentent ensemble un méthylènedioxy ou un éthylènedioxy ou bien l'un des radicaux $R^1$, $R^2$ et $R^3$ est un mono- ou un di-(alcoyle en $C_1$ à $C_4$)-amino et les deux autres sont des atomes d'hydrogène.

2. Composés selon la revendication 1, où X est un hydrogène.

3. Composés selon l'une des revendications 1 ou 2, où Y est un soufre.

4. Composés selon l'une des revendications 1, 2 ou 3, où Z est un phényle, un p-(fluoro ou méthoxy)phényle, un 3,4,5-triméthoxyphényle, un 3,4-méthylènedioxyphényle ou un 2-thiényle.

5. 2-Phényl-4,5,7,8-tétrahydro-4,4,8,8-tétraméthyl-1-H-thiépino[4,5-d]imidazole.

6. 2-p-Fluorophényl-4,5,7,8-tétrahydro-4,4,8,8-tétraméthyl-1-H-(thiépino[4,5-d]imidazole.

7. Les composés
2-(p-méthoxyphényl)-4,5,7,8-tétrahydro-4,4,8,8-tétraméthyl-1-H-thiépino[4,5-d]imidazole;
2-(m-méthoxyphényl)-4,5,7,8-tétrahydro-4,4,8,8-tétraméthyl-1-H-thiépino[4,5-d]imidazole;
2-(o-méthoxyphényl)-4,5,7,8-tétrahydro-4,4,8,8-tétraméthyl-1-H-thiépino[4,5-d]imidazole;
2-(3,4-diméthoxyphényl)-4,5,7,8-tétrahydro-4,4,8,8-tétraméthyl-1-H-thiépino[4,5-d]imidazole;
4,5,7,8-tétrahydro-4,4,8,8-tétraméthyl-2-(2,4,5-triméthoxyphényl)-1-H-thiépino[4,5-d]imidazole;
4,5,7,8-tétrahydro-4,4,8,8-tétraméthyl-2-(2,4,6-triméthoxyphényl)-1-H-thiépino[4,5-d]imidazole;
4,5,7,8-tétrahydro-4,4,8,8-tétraméthyl-2-(3,4,5-triméthoxyphényl)-1-H-thiépino[4,5-d]imidazole;
2-(m-méthoxyphényl)-4,5,7,8-tétrahydro-4,4,8,8-tétraméthyl-1-H-thiépino[4,5-d]imidazol-6,6-dioxyde;
2-(m-méthoxyphényl)-4,5,7,8-tétrahydro-4,4,8,8-tétraméthyl-1-H-thiépino[4,5-d]imidazol-6-oxyde.

8. Les composés
4,5,7,8-tétrahydro-4,4,8,8-tétraméthyl-2-p-tolyl-1-H-thiépino[4,5-d]imidazole;
4,5,7,8-tétrahydro-4,4,8,8-tétraméthyl-2-m-tolyl-1-H-thiépino[4,5-d]imidazole;
4,5,7,8-tétrahydro-4,4,8,8-tétraméthyl-2-o-tolyl-1-H-thiépino[4,5-d]imidazole;
2-(4,6-diméthylphényl)-4,5,7,8-tétrahydro-4,4,8,8-tétraméthyl-1-H-thiépino[4,5-d]imidazole;
2-(3,4-diméthylphényl)-4,5,7,8-tétrahydro-4,4,8,8-tétraméthyl-1-H-thiépino[4,5-d]imidazole.

9. Les composés
p-(4,5,7,8-tétrahydro-4,4,8,8-tétraméthyl-1-H-thiépino[4,5-d]imidazol-2-yl)phénol;
m-(4,5,7,8-tétrahydro-4,4,8,8-tétraméthyl-1-H-thiépino[4,5-d]imidazol-2-yl)phénol;
o-(4,5,7,8-tétrahydro-4,4,8,8-tétraméthyl-1-H-thiépino[4,5-d]imidazol-2-yl)phénol.

10. Les composés
2-(3,4-méthylènedioxyphényl)-4,5,7,8-tétrahydro-4,4,8,8-tétraméthyl-1-H-thiépino[4,5-d]-imidazole;
2-(3,4-éthylènedioxyphényl)-4,5,7,8-tétrahydro-4,4,8,8-tétraméthyl-1-H-thiépino[4,5-d]-imidazole;
2-(5-méthoxy-3,4-méthylènedioxyphényl)-4,5,7,8-tétrahydro-4,4,8,8-tétraméthyl-1-H-thiépino[4,5-d]imidazole.

11. Les composés
2-(m-fluorophényl)-4,5,7,8-tétrahydro-4,4,8,8-tétraméthyl-1-H-thiépino[4,5-d]imidazole;
2-(p-fluorophényl)-4,5,7,8-tétrahydro-4,4,8,8-tétraméthyl-1-H-thiépino[4,5-d]imidazole.

12. Les composés
2-phényl-4,5,7,8-tétrahydro-4,4,8,8-tétraméthyl-1-H-thiépino[4,5-d]imidazol-6,6-dioxyde;
2-phényl-4,5,7,8-tétrahydro-4,4,8,8-tétraméthyl-1-H-thiépino[4,5-d]imidazol-6-oxyde;
2-phényl-4,5,7,8-tétrahydro-1,4,4,8,8-pentaméthyl-1-H-thiépino[4,5-d]imidazole;
2-phényl-4,5,7,8-tétrahydro-1,4,4,8,8-pentaméthyl-1-H-thiépino[4,5-d]imidazol-6-oxyde;
1-n-butyl-2-phényl-4,5,7,8-tétrahydro-4,4,8,8-tétraméthyl-1-H-thiépino[4,5-d]imidazole.

13. 2-[p-(Diéthylamino)phényl]-4,5,7,8-tétrahydro-4,4,8,8-tétraméthyl-1-H-thiépino-[4,5-d]imidazole.

14. 2-(p-Méthylthiophényl)-4,5,7,8-tétrahydro-4,4,8,8-tétraméthyl-1-H-thiépino[4,5-d]-imidazole.

15. 4,5,7,8-Tétrahydro-4,4,8,8-tétraméthyl-2-(2-thiényl)-1-H-thiépino[4,5-d]imidazole.

16. Composé selon l'une des revendications 1-15 comme substance active pharmaceutique.

17. Composé selon l'une des revendications 1-15 comme substance active inhibant l'agglutination des plaquettes sanguines.

18. Composé selon l'une des revendications 1-15 comme substance active antihyperglycémique.

19. Médicament contenant un composé selon l'une des revendications 1-15.

20. Agent pour inhiber l'agglutination des plaquettes sanguines, contenant un composé selon l'une des revendications 1-15.

21. Agent antihyperglycémique contenant un composé selon l'une des revendications 1-15.

22. Procédé de préparation de composés de formule (I) de la revendication 1 et de leurs sels d'addition d'acides physiologiquement acceptables, caractérisé en ce que pour préparer un composé de formule (I) où X est un hydrogène, on fait réagir une dicétone de formule:

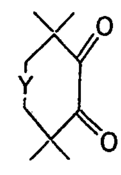

(II)

où Y a la signification donnée ci-dessus, avec un aldéhyde de formule:

$$Z-CH=O \quad (III)$$

où Z a la signification donnée ci-dessus, en présence d'un solvant polaire contenant des ions ammonium; en ce que pour préparer un

composé de formule (I), où X est un alcoyle en $C_1$ à $C_4$, on alcoyle un composé de formule (I) où X est un hydrogène; si on le désire en ce qu'on oxyde un composé de formule (I) où Y est un soufre en un composé de formule (I) où Y est un sulfinyle, et, si on le désire, en ce qu'on transforme un composé de formule (I) en un sel d'addition d'acide physiologiquement acceptable.

**Revendications pour l'Etat contractant:** AT

1. Procédé de préparation de dérivés d'imidazole de formule:

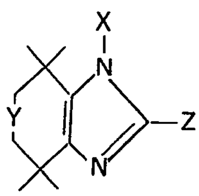

(I)

où X représente un hydrogène ou un alcoyle en $C_1$ à $C_4$, Y représente un soufre ou un groupe sulfinyle ou sulfonyle et Z un thiényle éventuellement substitué par un groupe méthyle ou un atome de fluor, ou un groupe de formule:

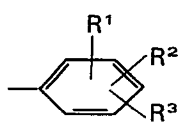

Z

où $R^1$, $R^2$ et $R^3$ représentent un hydrogène, un méthyle, un fluor, un hydroxy, un méthoxy, un méthylthio, ou bien deux radicaux $R^1$, $R^2$ ou $R^3$ voisins représentent ensemble un méthylènedioxy ou un éthylènedioxy ou bien l'un des radicaux $R^1$, $R^2$ et $R^3$ est un mono- ou un di-(alcoyle en $C_1$ à $C_4$)-amino et les deux autres sont des atomes d'hydrogène, et leurs sels d'addition d'acides physiologiquement acceptables, caractérisé en ce que, pour préparer un composé de formule (I) où X est un hydrogène, on fait réagir une dicétone de formule:

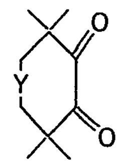

(II)

où Y a la signification donnée ci-dessus, avec un aldéhyde de formule:

$$Z-CH=O \qquad (III)$$

où Z a la signification donnée ci-dessus, en présence d'un solvant polaire contenant des ions ammonium; en ce que pour préparer un composé de formule (I), où X est un alcoyle en $C_1$ à $C_4$, on alcoyle un composé de formule (I) où X est un hydrogène; si on le désire en ce qu'on oxyde un composé de formule (I) où Y est un soufre en un composé de formule (I) où Y est un sulfinyle, et, si on le désire, en ce qu'on transforme un composé de formule (I) en un sel d'addition d'acide physiologiquement acceptable.

2. Procédé selon la revendication 1, caractérisé en ce qu'on utilise comme source d'ions ammonium un sel d'ammonium.

3. Procédé selon l'une des revendications 1 ou 2, caractérisé en ce qu'on prépare des composés de formule (I) où X est un hydrogène.

4. Procédé selon l'une des revendications 1, 2 ou 3, caractérisé en ce qu'on prépare des composés de formule (I) où Y est un soufre.

5. Procédé selon l'une des revendications 1-4, caractérisé en ce qu'on prépare des composés de formule (I) où Z est un phényle, un p-(fluoro ou méthoxy)phényle, un 3,4,5-triméthoxyphényle, un 3,4-méthylènedioxyphényle ou un 2-thiényle.

6. Procédé selon la revendication 5, caractérisé en ce qu'on prépare le 2-phényl-4,5,7,8-tétrahydro-4,4,8,8-tétraméthyl-1-H-thiépino[4,5-d]imidazole.

7. Procédé selon la revendication 5, caractérisé en ce qu'on prépare le 2-p-fluorophényl-4,5,7,8-tétrahydro-4,4,8,8-tétraméthyl-1-H-thiépino[4,5-d]imidazole.